Europäisches Patentamt

European Patent Office

Office européen des brevets

(11) Veröffentlichungsnummer: **0 012 817**
A1

(12) # EUROPÄISCHE PATENTANMELDUNG

(21) Anmeldenummer: 79104331.8

(22) Anmeldetag: 06.11.79

(51) Int. Cl.³: **A 61 B 1/24**

(30) Priorität: 27.12.78 IT 3132078

(43) Veröffentlichungstag der Anmeldung:
09.07.80 Patentblatt 80/14

(84) Benannte Vertragsstaaten:
CH DE FR

(71) Anmelder: Ferrari Agradi, Ulderico
Via Croce, 11
I-29100 - Piacenza(IT)

(72) Erfinder: Ferrari Agradi, Ulderico
Via Croce, 11
I-29100 - Piacenza(IT)

(74) Vertreter: Racheli, Adele, Ing.
Via San Michele del Carso, 4
I-20144 Mailand(IT)

(54) **Mehrfachreflektionshandgerät, das dem Benutzer die Sichtbarmachung von Körperteilen ermöglicht, die nicht unmittelbar sichtbar sind (beispielsweise das innere Ohr, der Hals usw.).**

(57) Die vorliegende Erfindung betrifft ein Mehrfachreflektionshandgerät, das dem Benutzer die Sichtbarkeit von normalerweise nicht sichtbaren Körperteilen ermöglicht. Das Gerät besteht aus einem Etui oder einer Kassette mit einem Scharnierdeckel (11) und weist mindestens zwei Spiegel (12,20) auf. Der erste Spiegel ist an einer einsteckbaren oder festschraubbaren Stange (17) befestigt, die ihrerseits am Ende eines biegsamen Schwenkarmes (15) befestigt ist, der durch ein Kugelgelenk (14) an der Kassettenbasis (10) angebracht ist. Durch die Anordnung der Spiegelträgerstange (17) kann der erste Spiegel, (20) der von einer am Ende des Schwenkarmes (15) eingeschraubten Lampe (21) beleuchtet wird, eine Neigung von ca. 10° gegenüber der Stange (17) einnehmen.

FIG. 2

EP 0 012 817 A1

Anmelder:

Ulderico FERRARI AGRADI

Via Croce, 11

I- 29100 PIACENZA

Mehrfachreflektionshandgerät, das dem Benutzer die Sichtbarmachung von Körperteilen ermöglicht, die nicht unmittelbar sichtbar sind (beispielsweise das innere d...,

der Hals usw.).

Die vorliegende Erfindung betrifft ein Mehrfachreflexhandgerät, das dem Benutzer die Sichtbarkeit von ...

teilen, die er nicht direkt sehen kann, wie beispiels...

das Innere des Ohrs, den Hals, Nacken, den Rücken usw.

möglich macht.

Die Schwierigkeiten sind bekannt, die man hat, Körperteile

zu untersuchen, die selbst mit Hilfe eines gewöhnlichen

Spiegels nicht direkt sichtbar sind. Da aber eine solche

Untersuchung oft aus hygienisch-sanitären Gründen nötig

ist, ist man gezwungen, die Hilfe des Nächsten in Anspruch

zu nehmen, der jedoch nicht immer bereit und verfügbar ...

- 2 -

Das erfindungsgemässe Gerät sieht vor, dem Benutzer die Möglichkeit zu geben, allein fast alle Teile des eigenen Körpers mit grösster Leichtigkeit zu besichtigen. Es besteht aus einem ersten Spiegel, der von einer Lichtquelle beleuchtet und am Ende eines schwenkbaren Armes angebracht ist und wenigstens einem zweiten Spiegel, der zum ersten unterschiedlich orientiert werden kann.

Um die Sichtbarkeit seitens des Benutzers zu erhöhen, kann das Gerät mit einem dritten Spiegel ausgestattet werden, der zum zweiten winkelmässig orientierbar ist.

Die Erfindung wird nachstehend anhand der beigefügten Zeichnungen, die nur als Beispiel dienen und nicht einschränkend sind, beschrieben. Es zeigen:

Fig. 1 eine Draufsicht auf das in einem offenen Behälter angeordnete Gerät,

Fig. 2 eine Schnittansicht gemäss der Linie II-II in Fig. 1 mit erhobenem Spiegelträgerarm,

Fig. 3a und 3b schematische Darstellungen des Verlaufs des Lichtstrahls in zwei verschiedenen Fällen.

In der Figuren sind die gleichen Elemente mit den gleichen Bezugsnummern angegeben.

Die in der Zeichnung dargestellte besondere Verwirklichungsform stellt ein erfindungsgemässes Gerät dar, das mit einem

beleuchteten Spiegel und zwei zusätzlichen Spiegeln ausgestattet ist.

Die Figuren zeigen ein in einem Behälter angeordnetes Mehrfachreflektionsgerät.

Der Behälter besteht aus einer Kassette mit rechteckiger Basis 10 mit einem Scharnierdeckel 11. Auf dem Boden der beiden Elemente 10, 11 sind zwei Spiegel 12, 13 angeordnet. In einer Ecke der Basis 10 ist ein Kugelgelenk 14 befestigt, auf dem ein biegsamer Arm 15 lagert.

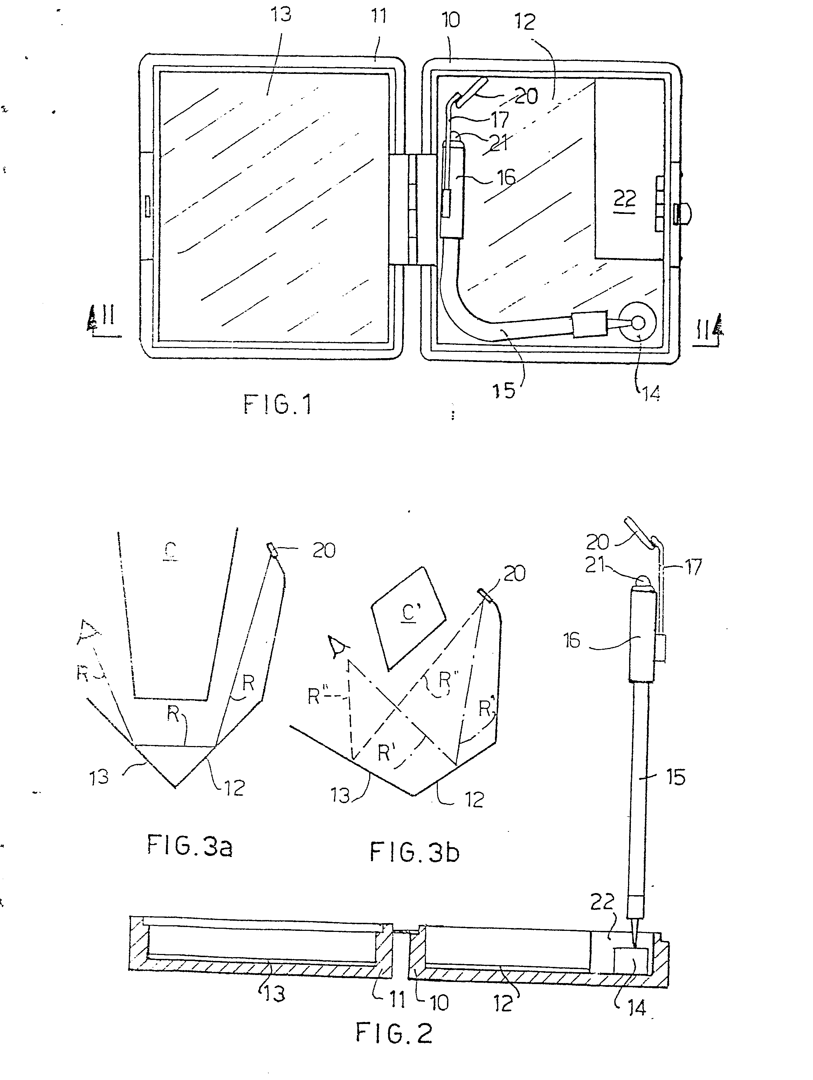

Am Ende des biegsamen Armes 15 ist mit bekannten Mitteln, beispielsweise eine Feder oder Einsteckvorrichtung, ein Spiegel 20 mit Spiegelhalter 16 befestigt. Der Spiegelhalter 16 besteht im wesentlichen aus einer einsteckbaren oder mit einer Schraube regelbaren Stange 17 (Fig. 2), die so angeordnet ist, dass der Spiegel 20 gegenüber der Stange 17 eine Neigung von ca. 10° annimmt.

Im Gewindeteil 16 des Arms 15 ist eine (gegebenenfalls linsenförmige) Lampe 21 vorgesehen, die elektronisch oder elektrisch, beispielsweise von einer oder mehreren in einem Etui 22 angeordneten Batterien gespeist wird.

Das von der Lampe 21 ausgestrahlte Lichtbündel muss auf den waagrecht regelbaren Spiegel 20 und auf den zu untersuchenden Punkt gerichtet sein, dessen Bild eingefangen und reflektiert

und gegebenenfalls vom Spiegel 20 vergrössert wird, der auf die beiden anderen Spiegel 12, 13 reflektiert und dadurch dem Benutzer die Sichtbarkeit von Teilen ermöglicht, die sonst nicht sichtbar wären.

Der biegsame Arm 15 kann eine beliebige Stellung infolge des Kugelgelenkes 14 annehmen.

Dadurch kann der Spiegel 20 jede beliebige Stellung gegenüber den Spiegeln 12 und 13 annehmen, die so angeordnet sind, dass sie einen zweckmässigen Winkel bilden, um dem Benutzer die Sichtbarkeit des infrage kommenden Teiles zu ermöglichen. Der Lichtstrahlengang kann somit einen Spiegel 12 oder 13 wie auch beide Spiegel treffen, wie aus den Figuren 3a und 3b ersichtlich ist. Aus Fig. 3a ist ersichtlich, wie ein grosser Teil C des menschlichen Körpers mit der zu untersuchenden Stelle interferieren kann, wenn der Lichtstrahlengang der mit R bezeichnete ist. In Fig. 3b ist ein kürzerer Strahlengang R' und R" dargestellt, der verwendet werden kann, wenn der nicht unmittelbar sichtbare Körperteil C' geringere Ausmasse hat.

Der Spiegel 20 kann je nach Bedarf mit einem Spiegel unterschiedlicher Ausmasse ausgewechselt werden, beispielsweise mit einem grösseren zur Untersuchung der Hämorrhoiden. Ausserdem kann auch die Lampe in anderer als der in der Zeichnung dargestellten Art angebracht werden und zwar auf dem Spiegel 20.

Um das Gerät zusammenzulegen, wird der Spiegel abgeschraubt oder abgenommen und der biegsame Arm 15 im Innern der Basis 10 umgebogen, wie in Fig. 1 dargestellt.

5. Die Befestigung des Armes 15 im Etui kann auch mit anderen Mitteln als dem Kugelgelenk erfolgen und zwar beispielsweise mit einem Stift und entsprechendem Halter.

Der Arm 15 kann ausserdem ausgetauscht und mit üKntsprechenden Verlängerungen, die an ihren Enden mit Anschlüssen versehen sind, verlängert werden.

Das erfindungsgemässe Mehrfachreflektionsgerät kann in verschiedene tragbare Gegenstände oder Tischgeräte eingesetzt werden, beispielsweise in eine Brief-oder Schecktasche, in der ein- oder zwei Spiegel 12, 13 mit einer bleistiftartigen den beleuchteten Vergrösserungsspiegel tragenden Stange, die die Batterie oder die Batterien enthält, angeordnet werden. Das Gerät kann insbesondere auch in einen Kosmetikkoffer oder einen sogenannten 24-Stunden-Koffer eingesetzt werden.

In gleicher Weise kann das erfindungsgemässe Gerät in ein Merkbuch oder einen Terminkalender mit einem Etui oder einer Kassette angeordnet werden, worin alle erforderlichen Elemente enthalten sind.

0012817

Anmelder:

Ulderico FERRARI AGRADI

Via Croce, 11

I-29100 PIACENZA

Patentansprüche

1.      Mehrfachhandreflektionsgerät, das dem Benutzer
die Sichtbarkeit von Teilen seines eigenen Körpers ermöglicht,
die sonst nicht direkt sichtbar sind, dadurch gekennzeichnet,
dass es eine von elektrischen oder elektronischen Mitteln
gespeiste Lichtquelle (21), einen ersten Spiegel (20), der
das von dem Lichtbündel beleuchtete Bild reflektiert, und
mindestens einen zweiten zum ersten in verschiedener Weise
-chwenkbaren Spiegel (12) umfasst.

2.      Gerät nach Anspruch 1, dadurch gekennzeichnet,
dass es mit einem winkelmässig zum zweiten Spiegel (12)
beweglichen dritten Spiegel (13) versehen ist.

3.    Gerät nach Anspruch 1, dadurch gekennzeichnet, dass der Spiegel (20) ein (konkaver) Vergrösserungsspiegel oder ein normaler (flacher) Spiegel ist.

4.    Gerät nach Anspruch 1, dadurch gekennzeichnet, dass der Spiegel (20), der das von der Lampe (21) beleuchtete Bild reflektiert, am Ende eines Schwenkarms (15), der mit einem Kugelgelenk (14) an der Basis befestigt ist, angebracht ist.

5.    Gerät nach den Ansprüchen 1 bis 4, dadurch gekennzeichnet, dass der Schwenkarm (15) aus biegsamem Werkstoff besteht.

6.    Gerät nach Anspruch 1, dadurch gekennzeichnet, dass der durch die Einschraub- oder Einsteckstange (17) regelbare Spiegel (20) von einem Spiegelhalterelement (16) so abgestützt ist, dass er gegenüber der Stange (17) senkrecht um ca. 10° geneigt ist.

7.    Gerät nach Anspruch 1, dadurch gekennzeichnet, dass der Spiegelträgerarm (15) mit der Basis des Behälters (10) durch einen Stift oder ein Gelenk verbunden ist.

8.    Gerät nach Anspruch 1 dadurch gekennzeichnet, dass der Spiegelträgerarm (15) von verschiedenen austauschbaren und/oder verlängerbaren Elementen ersetzt werden kann.

9.      Gerät nach Anspruch 1, dadurch gekennzeichnet, dass es in einem Tisch- oder Taschenetui, in dem andere Gegenstände enthalten sind und für verschiedene Zwecke verwendet werden, enthalten sein kann.

FIG.1

FIG.3a

FIG.3b

FIG.2

Europäisches
Patentamt

## EUROPÄISCHER RECHERCHENBERICHT

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | FR - A - 2 380 763 (J.H. PETRAGUE) <br> * Seite 1, Zeilen 15-20; Seite 3, Zeile 15 - Seite 4, Zeile 18; Abbildungen 2,3 * <br> -- | 1,3,7 | A 61 B 1/24 |
| X | FR - A - 723 336 (M. FAGEOT) <br> * Seite 1, Zeilen 18-36; Seite 1, Zeile 56 - Seite 3, Zeile 12; Abbildungen 1-4 * <br> -- | 1,3,4 7-9 | |
| | FR - A - 2 370 459 (H. HAKUBA) <br> * Seite 2, Zeilen 15-30; Seite 3, Zeilen 3-32; Seite 7, Zeile 21 - Seite 8, Zeile 22; Seite 10, Zeile 14 - Seite 12, Zeile 35; Seite 13, Zeilen 2-24; Seite 20, Zeile 32 - Seite 23, Zeile 6; Abbildungen 5-15,22,23,42-45 * <br> -- | 1,3,4 6-9 | **RECHERCHIERTE SACHGEBIETE (Int. Cl. 3)** <br><br> A 61 B 1/24 |
| X | FR - A - 1 556 983 (M. VINOT) <br> * Seite 1, linke Spalte, Zeile 39 - rechte Spalte, Zeile 40; Seite 2, linke Spalte, Zeile 6 - rechte Spalte, Zeile 57; Abbildungen 1,2 * <br> -- | 1,3,7 | |
| X | CH - A - 188 730 (H. VAN HARTINGS-VELT) <br> * Seite 1, rechte Spalte, Zeile 5 - Seite 3, rechte Spalte, Zeile 3; Seite 4, linke Spalte, Zeilen 9-34; Abbildungen 1-5 * <br> -- ./. | 1-3 | **KATEGORIE DER GENANNTEN DOKUMENTE** <br><br> X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument <br> &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 26-03-1980 | RIEB |

EPA form 1503.1 06.78

## EUROPÄISCHER RECHERCHENBERICHT

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | <u>FR - A - 438 362</u> (J.F. GLOVER)<br><br>* Seite 1, Zeilen 44-51; Abbildung 1 *<br><br>-- | 2,3 | |
| | <u>DE - A - 2 020 997</u> (U. THOMAS)<br><br>* Seite 2, Zeilen 4-12; Seite 3, Zeilen 5-17; Seite 5, Zeile 24 - Seite 6, Zeile 11; Seite 9, Zeile 2 - Seite 10, Zeile 3; Seite 12, Zeile 18 - Seite 14, Zeile 3; Abbildungen 2,4, 5,9 *<br><br>-- | 3-5,7, 8 | RECHERCHIERTE SACHGEBIETE (Int. Cl.) |
| | <u>FR - A - 896 217</u> (C. BRUCKER)<br><br>* Seite 1, Zeile 12 - Seite 2, Zeile 2; Seite 2, Zeilen 35-73; Seite 3, Zeilen 50-70; Abbildungen 1,2,5,6 *<br><br>---- | 3,7-9 | |